# EUROPÄISCHE PATENTSCHRIFT

(11) **EP 0 598 964 B1**
(45) Veröffentlichungstag und Bekanntmachung des Hinweises auf die Patenterteilung: **07.07.1999**
(21) Anmeldenummer: 92810905.7
(22) Anmeldetag: 20.11.1992
(51) Int. Cl.: A61F 2/46

(54) **Körper zum Verteilen von Knochenzement für die Verankerung von Implantaten**
Bone cement dispenser body for implant fixation
Corps pour dispenser du ciment osseux pour la fixation d'implants

(43) Veröffentlichungstag der Anmeldung: 01.06.1994
(73) Patentinhaber: Sulzer Orthopädie AG, 6340 Baar (CH)
(72) Erfinder: Willert, Hans Georg, Prof. Dr., W-3400 Göttingen (DE); Bider, Kurt, CH-8400 Winterthur (CH)
(74) Vertreter: Sulzer Management AG

(56) Entgegenhaltungen:
- EP-A- 0 073 604
- WO-A-90/04953
- CH-A- 639 549

## Beschreibung

Die Erfindung handelt von einem Körper zum Verteilen von Knochenzement für die Verankerung von Implantaten in einer vorbereiteten und der Aussengeometrie des Implantats entsprechenden Knochenaushöhlung, wobei der Körper im Bereich seiner an die Knochenaushöhlung ansetzbaren Randfläche aus elastischem Kunststoff besteht, der dichtend an die Knochenaushöhlung anpassbar ist, und wobei an den Körper eine Knochenzementspritze anschliessbar ist und der Körper einen Durchgang zu einem zwischen Knochenaushöhlung und Körper einschliessbaren Volumen aufweist.

Aus der Patentschrift CH 639549 ist eine auf eine Zementspritze aufsetzbare Kappe bekannt, welche in einem elastischen, konischen Mundstück endet, um dieses beim Einspritzen des Zementes am Aussenrand einer Knochenhöhlung anzupressen. Ein Nachteil dieser Anordnung ist, dass grosse Mengen des Zements, der zeitabhängig aushärtet und sich mengenabhängig erwärmt, in die Knochenaushöhlung hineinbewegt wird, um ein Verpressen zu erreichen und dass anschliessend beim Einsetzen des Implantats wieder grössere Mengen an Zement aus der Knochenaushöhlung in das umgebende Operationsfeld herausgedrückt werden. Eine andere Möglichkeit besteht in der Verwendung von hohlen Implantaten mit einem von aussen anspeisbaren Verteilsystem wie es in der Patentschrift US 4,274,163 beschrieben wird. Ein Nachteil dieser Anordnung besteht darin, dass eine Schwächung des Implantats durch das Verteilsystem entsteht, dass im Verhältnis zu den relativ kleinen Durchtrittsquerschnitten lange Fliesswege mit zusammenfliessenden Oberflächen entstehen, und dass die Herstellung der Implantate durch ein derartig intergriertes Verteilsystem verteuert wird.

Diesen Umständen trägt die Erfindung Rechnung. Sie hat die Aufgabe ohne Beeinträchtigung der Struktur des Implantats den Knochenzement gegenüber der Knochenaushöhlung in seine endgültige Lage zu bringen. Sie löst diese Aufgabe mit den Kennzeichen des unabhängigen Patentanspruchs 1.

Die Erfindung hat den Vorteil, dass grosse Querschnitte zum Anspeisen des Zwischenraums zwischen Implantat und Knochenaushöhlung möglich sind, dass nur soviel Knochenzement, wie zum Aushärten und Verbinden notwendig ist, in die Knochenaushöhlung eingebracht wird, und dass wegen der grossen Anspeisquerschnitte ein vorgesehener Anpressdruck beim Füllen der Knochenaushöhlung sehr genau einhaltbar ist.

Ein Körper zum Verteilen von Knochenzement liegt mit einem elastischen Kragen am äusseren Rand einer Knochenaushöhlung an und besitzt einen Durchgang zur Knochenaushöhlung hin, in dem eine Spritze für Knochenzement einsetzbar ist. Der Körper ist in der Knochenaushöhlung als Verdrängerkörper mit der Form des später einzusetzenden Implantats ausgeführt. Beim Ausspritzen des Zwischenraums zwischen Körper und Knochenaushöhlung wird ein vorgesehener Druck solange gehalten, bis die Zähigkeit vom Knochenzement ausreicht, um den Körper ohne Rückfliessen von Knochenzement aus der Knochenaushöhlung herauszunehmen. Anschliessend kann das eigentliche Implantat in der so vorbereiteten, immer noch plastisch verformbaren Gegenform aus Knochenzement eingepresst werden.

In den Unteransprüchen 2 bis 5 sind vorteilhafte Weiterbildungen der Erfindung gezeigt. Im folgenden wird die Erfindung anhand von Ausführungsbeispielen beschrieben. Es zeigen:
- Fig.1:: Einen Längsschnitt durch einen in einer Knochenaushöhlung eingesetzten, für eine Hüftgelenkpfanne vorgesehenen Körper nach dem Einspritzen von Knochenzement;
- Fig.2:: Eine Draufsicht auf den Körper in Figur 1;
- Fig.3:: Einen Längsschnitt entsprechend der Anordnung in Figur 1 vor dem Einspritzen von Knochenzement, bei der der Körper mit einem Kragen auf der Stirnfläche zur Knochenaushöhlung aufliegt und mit Nocken innerhalb der Knochenaushöhlung zentriert ist, und
- Fig.4:: Eine Draufsicht auf den Körper in Figur 3.

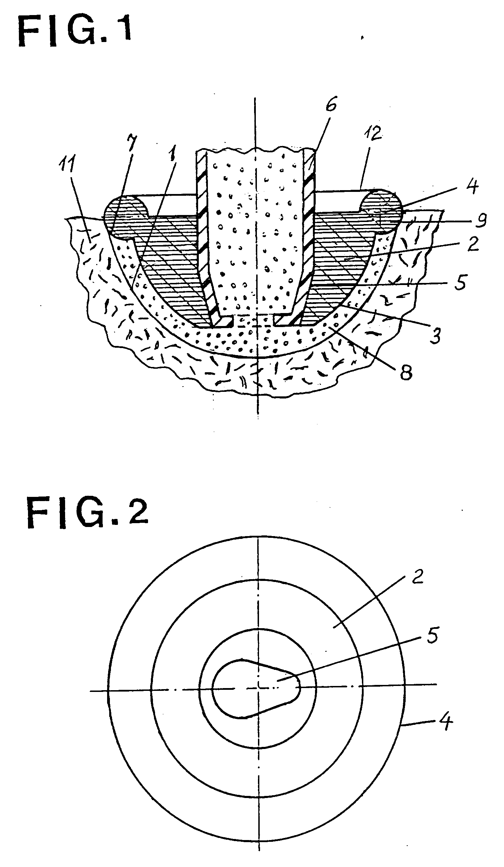

In den Figuren 1 und 2 liegt ein Körper 2 mit einem Kragen 4 als Wulst 12 aus elastischem Kunststoff 7 wie zum Beispiel aus Silikonkautschuk 9 am Rand einer Knochenaushöhlung 1 auf. Die in die Knochenaushöhlung 1 hineinragende Form 3 entspricht der einer später einzusetzenden Hüftgelenkschale. Der Körper 2 besitzt einen Durchgang 5 an den eine Knochenzementpsritze 6 angepresst ist, um den hydrostatischen Auftrieb beim Einspressen von Knochenzement 8 zu kompensieren. Durch den grossen Querschnitt vom Durchgang 5 und durch die kurzen Fliesswege ergeben sich kaum Druckverluste, bis der Knochenzement den Zwischenraum zwischen Form 3 und der Knochenaushöhlung 1 erreicht hat.

Ein als optimal erachteter Druck, bei dem das Knochengewebe 11 der Knochenaushöhlung 1 mit Knochenzement 8 nicht überladen wird, kann sehr genau eingehalten werden. Wenn der Knochenzement gemäss Zeitplan eine Zähigkeit erreicht hat, bei der er unter Schwerkraft in dünnen Schichten wie im Zwischenraum zwischen der Form 3 und der Knochenaushöhlung nicht mehr fliesst, kann der Körper 2 herausgelöst werden. Dabei hat sich gezeigt, dass ein Trennmittel wie zum Beispiel eine physiologische Kochsalzlösung von Vorteil ist, wenn es vor dem Einspritzen des Knochenzements 8 am Körper 2 aufgebracht wurde.

Nach dem Herauslösen der Form 3 wird das eigentliche Implantat in den immer noch plastisch verformbaren Knochenzement eingepresst. Ein Überladen des Knochengewebes 11 mit Knochenzement 8 ist wegen dessen feiner Mikrostruktur und der nun höheren Zähigkeit des Knochenzements kaum noch möglich.

Der Körper 2 ist wiederverwendbar. Er muss aber soweit temperaturstabil sein, dass er wie zum Beispiel Silikonkautschuk bei 134°C dampfsterilisierbar ist.

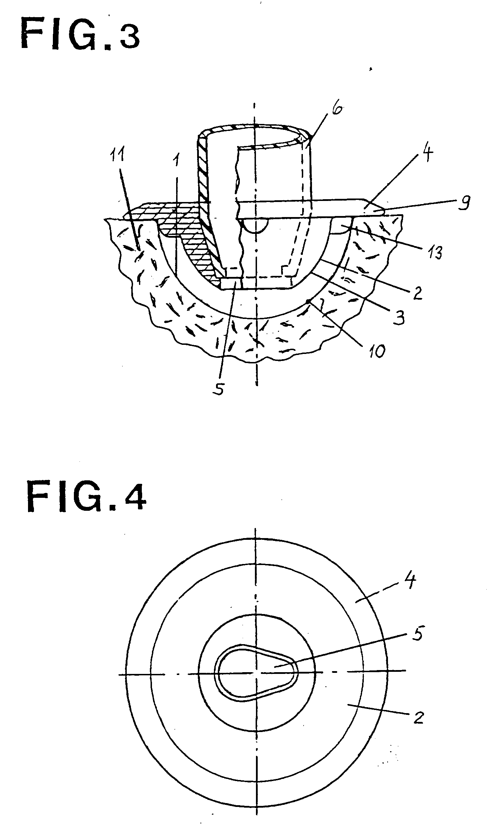

In den Figuren 3 und 4 ist ein Körper 2 für eine Hüftgelenkpfanne auf einer vorbereiteten Knochenaushöhlung 1 aufgesetzt und stützt sich mit einem seitlich über die Knochenaushöhlung vorstehenden Kragen 4 auf dem äusseren Knochengewebe ab. Der dichtende Teil des Kragens 4 ist mit Silikonkautschuk 4 belegt, um Unebenheiten am Knochengewebe auszugleichen. Die Dichtwirkung ist soweit begrenzt, dass die eingeschlossene Luft beim Einspritzen des Knochenzementes entweichen kann. Zur besseren Zentrierung des Körpers 2 sind unter dem Kragen 4 Nocken 13 angebracht, welche sich innerhalb der Knochenaushöhlung 1 am Knochengewebe 11 abstützen.

Das Verteilen von Knochenzement 8 in einer Knochenaushöhlung 1 mit einem Körper 2 wie er in den Ansprüchen 1 bis 5 beschrieben ist, kann schrittweise vorgenommen werden, indem
- in einem ersten Schritt der Körper 2, der der Aussenform des einzusetzenden Implantats nachgebildet ist mit einem Kragen 4 an die Knochenaushöhlung 1 angepresst wird,
- in einem zweiten Schritt Knochenzement 8 über einen Durchgang 5 am Körper 2 eingespritzt wird und unter Nachpressen bis in die Mikrostruktur der Oberfläche 10 der Knochenaushöhlung 1 verteilt wird,
- in einem dritten Schritt der Körper 2 aus der Knochenaushöhlung 1 entfernt wird, wobei der Knochenzement 8 im Hinblick auf das eigentliche Implantat vorverformt in der Körperaushöhlung 1 verbleibt,
- in einem vierten Schritt das eigentliche Implantat in die Knochenaushöhlung 1 eingesetzt wird und der überflüssige am Rand hervorquellende Knochenzement 8 entfernt wird, und
- in einem fünften Schritt das Implantat in einer ausgerichteten Position gehalten wird, bis der Knochenzement 8 ausgehärtet ist.

Weiterhin ist es möglich, den Körper 2 vor dem ersten Schritt mit einem Trennmittel zu benetzen.

Als Trennmittel kann eine sterile physiologische Kochsalzlösung verwendet werden.

## Patentansprüche

1. Körper (2) zum Verteilen von Knochenzement (8) für die Verankerung von Implantaten in einer vorbereiteten und der Aussengeometrie des Implantats entsprechenden Knochenaushöhlung (1), wobei der Körper einen Kragen (4) aufweist, der an die Knochenaushöhlung ansetzbar ist und aus elastischem Kunststoff besteht, der dichtend an die Knochenaushöhlung anpassbar ist, und wobei an den Körper eine Knochenzementspritze anschliessbar ist und der Körper (2) einen Durchgang (5) zu einem zwischen Knochenaushöhlung und Körper einschliessbaren Volumen aufweist, dadurch gekennzeichnet, dass der Körper (2) so geformt ist, dass er in die Knochenaushöhlung einsetzbar ist, wobei er der Aussenform des einzusetzenden Implantats nachgebildet ist, und dass ein einer gewünschten Zementmenge entsprechender Zwischenraum zwischen dem Körper (2) und der Knochenaushöhlung (1) herstellbar ist.

2. Körper (2) nach Anspruch (1), dadurch gekennzeichnet, dass er der Aussenform einer Hüftgelenkschale nachgebildet ist.

3. Körper (2) nach einem der Ansprüche 1 oder 2, dadurch gekennzeichnet, dass der Kunststoff (7) soweit temperaturstabil ist, dass er bei 134°C dampfsterilisierbar ist.

4. Körper (2) nach einem der Ansprüche 1 oder 2, dadurch gekennzeichnet, dass mindestens seine Oberfläche im Bereich des Kragens (4) aus Silikonkautschuk (9) besteht.

5. Körper (2) nach einem der Ansprüche 2 bis 4, dadurch gekennzeichnet, dass er unter dem Kragen (4) Nocken (13) aufweist, mit denen er an der Knochenaushöhlung (1) zentrierbar ist.

## Claims

1. A body (2) for the distribution of bone cement (8) for anchoring implants in a hollow (1) prepared in bone to correspond with the outer geometry of the implant, where the body exhibits a collar (4) which may be set against the hollow in the bone and consists of elastic plastics able to fit against the hollow in the bone to make a seal, and where a nozzle for bone cement may be connected to the body, the body (2) exhibiting a way through (5) to a volume which may be enclosed between the hollow in the bone and the body, characterized in that
the body (2) is so shaped that it may be inserted in the hollow in the bone, being formed to the outer shape of the implant which is to be inserted, and that a space may be produced between the body (2) and the hollow (1) in the bone to correspond with a desired amount of cement.

2. A body (2) as in Claim 1, characterized in that it is formed to the outer shape of a hipjoint shell.

3. A body (2) as in one of the Claims 1 or 2, characterized in that the plastics (7) is so far temperature-stable that it is vapour-sterilizable at 134°C.

4. A body (2) as in one of the Claims 1 or 2, characterized in that at least its surface in the region of the collar (4) consists of silicone rubber (9).

5. A body (2) as in one of the Claims 2 to 4, characterized in that under the collar (4) it exhibits nodules (13) by which it may be centred in the hollow (1) in the bone .

## Revendications

1. Corps (2) pour distribuer du ciment osseux (8) pour l'ancrage d'implants dans une cavité osseuse préparée (1), correspondant à la géométrie extérieure de l'implant, où le corps présente un col (4) qui peut être appliqué à la cavité osseuse et qui est réalisé en matière synthétique élastique adaptable d'une manière étanche à la cavité osseuse, et où il peut être raccordé au corps une seringue d'injection de ciment osseux et le corps (2) présente un passage (5) menant à un volume pouvant être enfermé entre la cavité osseuse et le corps, caractérisé en ce que le corps (2) est formé de façon qu'il puisse être placé dans la cavité osseuse, où il est réalisé pour correspondre à la forme extérieure de l'implant à mettre en place et en ce qu'il peut être réalisé un espace intermédiaire correspondant à une quantité de ciment souhaitée entre le corps (2) et la cavité osseuse (1).

2. Corps (2) selon la revendication 1, caractérisé en ce qu'il est réalisé pour imiter la forme extérieure d'une cupule acétabulaire.

3. Corps (2) selon l'une des revendications 1 ou 2, caractérisé en ce que la matière synthétique (7) a une résistance thermique suffisante pour qu'elle puisse être stérilisée à la vapeur à 134°C.

4. Corps (2) selon l'une des revendications 1 ou 2, caractérisé en ce qu'au moins sa surface au voisinage du col (4) est réalisée en caoutchouc silicone (9).

5. Corps (2) selon l'une des revendications 2 à 4, caractérisé en ce qu'il présente sous le col (4) des cames (13) avec lesquelles il peut être centré à la cavité osseuse (1).
